(19) Europäisches Patentamt
European Patent Office
Office européen des brevets

(11) Publication number: 0 255 420 B1

# (12) EUROPEAN PATENT SPECIFICATION

(45) Date of publication of the patent specification: 27.12.90

(51) Int. Cl.[5]: A61K 31/70, C07H 15/256

(21) Application number: 87401589.4

(22) Date of filing: 07.07.87

## (54) Antiviral agent for inhibiting growth of virus of acquired immune deficiency syndrome (AIDS).

(30) Priority: 26.07.86 JP 174724/86

(43) Date of publication of application: 03.02.88 Bulletin 88/5

(45) Publication of the grant of the patent: 27.12.90 Bulletin 90/52

(84) Designated Contracting States: DE FR GB IT

(56) References cited:
EXPERIENTIA, vol. 36, 1980, page 304, Birkhäuser Verlag, Basel, CH; R. POMPEI et al.: "Antiviral activity of glycyrrhizic acid"
NATURE, vol. 281, 25th October 1979, pages 689-690, MacMillan Journals Ltd; R. POMPEI et al.: "Glycyrrhizic acid inhibits virus growth and inactivates virus particles"
CHEMICAL ABSTRACTS, vol. 107, no. 11, 14th September 1987, page 28, no. 89374y, Columbus, Ohio, US; M. ITO et al.: "Inhibitory effect of glycyrrhizin on the cytopathic activity of human immune deficiency virus"
CHEMICAL ABSTRACTS, vol. 107, no. 7, 17th August 1987, page 21, no. 51432c, Columbus, Ohio, US; M. ITO et al.: "Inhibitory effect of glycyrrhizin on the in vitro infectivity and cytopathic activity of the human immunodeficiency virus"

(73) Proprietor: MINOPHAGEN PHARMACEUTICAL COMPANY, 2-7, 3-chome, Yotsuya Shinjuku-ku, Tokyo(JP)

(72) Inventor: Ito, Masahiko, 74-1, Aza Shichishinomiya Watari, Fukushima-shi Fukushima-ken(JP)
Inventor: Nakashima, Hideki, 924-6 Ooaza Ogushi, Ube-shi Yamaguchi-ken(JP)
Inventor: Yamamoto, Naoki, 1-7-12 Higashikobayama-cho, Ube-shi Yamaguchi-ken(JP)
Inventor: Baba, Masanori, 8 Fukushima-idai Apartment House 2-3-47, Noda-machi Fukushima-shi Fukushima-ken(JP)
Inventor: Shigeta, Shiro, 147-28 Aza Kubouchi Oomori, Fukushima-shi Fukushima-ken(JP)

(74) Representative: Cournarie, Michèle et al, OFFICE BLETRY R.& G. ROGER-PETIT & R.BLETRY 2, boulevard de Strasbourg, F-75010 Paris(FR)

(56) References cited: (continuation)
CDC AIDS Weekly, 21 May 1988
Antiviral Research, vol. 11, 1989; pp. 255-262
Tohoku Journal Exp. Med., ed. 158, 1989; pp. 25-35
Antiviral Research, vol. 10, 1988; pp. 289-298

The file contains technical information submitted after the application was filed and not included in this specification

EP 0 255 420 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Acquired immune deficiency syndrome (AIDS) is a disease which was discovered in U.S.A. in 1982. Since AIDS is caused by virus, it is given a nomenclature such as Lymphadenopathy-associated virus (LAV) or Human T-lymphotropic retrovirus type III (HTLV-III), according to the respective discoverers. Yet it was generally known in terms of the combined name of HTLV-III/LAV, that is represented as human immunodeficiency virus (HIV) at the present time. Namely it is concerned with human retrovirus.

AIDS is such a disease that helper T cell of human lymphocytes are destroyed by retrovirus and results in opportunistic infections and malignancies, causing a high percent of deaths.

HIV is present in the blood, semen and saliva of the patient so that its infections occur through blood transfusion or some other causes.

There have heretofore been some reports as reviewed for instance in FDA Drug Bull., 15(3), 1985, 27–32, on remedies for this disease. As agents capable of controlling reverse transcriptase activity, important in the reproduction of retrovirus contained in HIV, there are raised HPA-23 etc, on one hand and on the other hand, as those capable of inhibiting DNA synthesis of retrovirus, there are raised 3'-azido-3'deoxythymidine, etc.

In addition, interleukin-II, interferons and isoprinosine are used as immuno-potentiators to relieve the patient of immune deficiency. However, at the present day, there are no agents to sufifciently ensure a therapeutic effect so that the exploitation of a more effective agent is considered very desirable.

In recent years, it has been found that glycyrrhizin, one of the active components of licorice, is reported as demonstrating antiviral activity in vitro against several kinds of DNA and RNA virus (Pompei R. et al; Nature 281, p. 689–690, 1972 and Experientia 36 (1980)304, Birkhäuser Verlag, Basel CH), but it has not yet been known whether glycyrrhizin can inhibit the growth of HIV as the cause of AIDS. Thus the present invention has been completed on the basis of discovery that glycyrrhizin can inhibit the growth of HIV and it has an action of immuno-potentiators.

### SUMMARY OF THE INVENTION:

One object of the present invention is to provide a therapeutic agent compound of glycyrrhizin as an effective substance for the treatment of patients of AIDS.

Another object of the invention is to provide a therapeutic agent containing an alkali-added salt of glycyrrhizin, pharmaceutically acceptable, as an effective substance for the treatment of patients of AIDS. Other objects of the invention will become apparent from the following detailed description and preferred embodiments of the invention.

### BRIEF DESCRIPTION OF THE DRAWINGS:

Fig. 1 is a view showing direct effect of glycyrrhizin on HIV induced plaque forming activity in MT-4 cells. Results are the mean of two experiments each performed in triplicate.

Fig. 2 is a view showing inhibition of cytopathic effect of HIV on MT-4 cells by glycyrrhizin. MT-4 cells ($3\times10^5$) were exposed to HIV ($6\times10^2$PFU) and cultured in the presence and absence of various concentrations of glycyrrhizin

(▨).

Control cells were similarly treated, but were not exposed to the virus

(□).

On day 3 in culture, the total viable cells were counted. Data are from a single experiment, representative of four separate experiments.

### DETAILED DESCRIPTION OF THE INVENTION:

The agent of the invention for inhibiting HIV is noted to have glycyrrhizin one of the effective components of licorice, as an effective substance represented by the following formula:

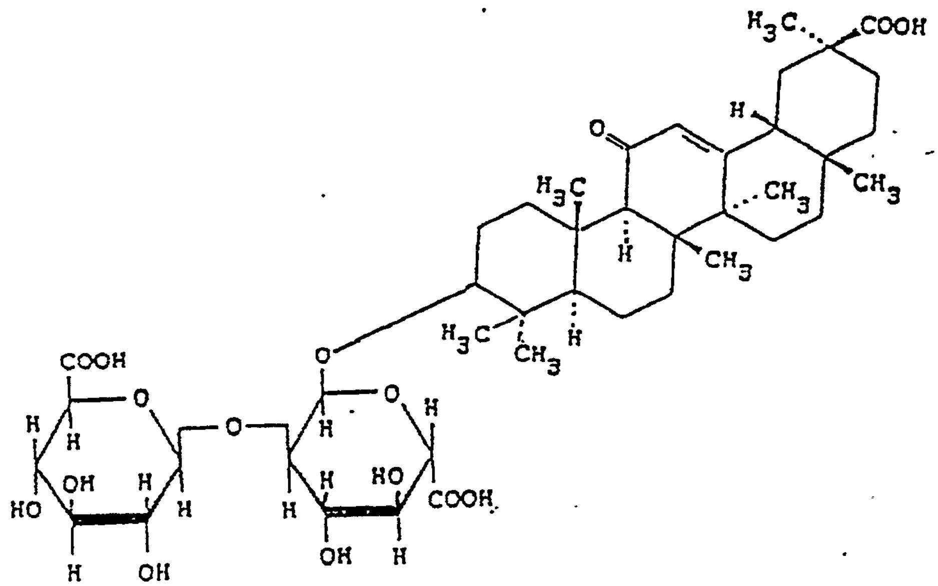

As shown in the following pharmacological data, glycyrrhizin has an outstanding effect of inhibiting the growth of HIV and moreover, it has an action of immuno-potentiators so that it is considered an effective therapeutic agent for the treatment of AIDS.

While glycyrrhizin can be offered as it is, so as to become an agent for inhibiting HIV, it is also possible to offer pharmaceutically acceptable alkali-added salts thereof, i.e. a salt of ammonium, alkalimetal or choline which are obtained by conjugating glycyrrhizin and inorganic or organic bases as effective substances for administration to patients of AIDS. Both oral and non-oral administrations are available as required. Doses may be generally within 200/400 mg/day for adults, depending on age and symptom until it proves effective as expected.

The forms of dose for administration may be powder, granules, tablets and capsules with which one or more kinds of excipients (carriers) pharmaceutically acceptable, such as lactose, potato starch, sodium alginate, aminoacetic acid, methionine or calcium carbonate are combined.

In the case of non-oral administration, doses of 120-300 mg per day for adults are given. Namely in the case of injection, the solvent may be distilled water or physiological saline solution alone or an amino acid (aminoacetic acid,cysteine and methionine) having an action of detoxication may be added.

By the way, agents of glycyrrhizin or recent date have shown excellent effects on the prevention and therapy of chronic viral hepatitis and even a large dose has witnessed no side effect at all (Suzuki et al, Medicine's progress 102, p.526-578, 1977).

Next, certain pharmaceutical examples of preparation of the agent of the present invention will b given as follows.

[Pharmaceutical example 1]

| [Pharmaceutical example 1] | |
|---|---|
| Manufacture of tablets: | |
| glycyrrhizin | 25 mg |
| potato starch | 220 mg |
| Magnesium stearate | 5 mg |
| Total | 250 mg |

Mixtures of the above-mentioned composition are formed into tablets by usual manner.

| [Pharmaceutical example 2] | |
|---|---|
| Manufacture of sugar-coated tablets: | |
| glycyrrhizin | 25 mg |
| glycine | 25 mg |
| methionine | 25 mg |
| calcium carbonate | suitable amount |
| lactose | suitable amount |
| carboxymethyl cellulose sodium | suitable amount |
| Total | 300 mg |

Mixtures of the above mentioned composition are formed into sugar-coated tablets in the usual manner.

[Pharmaceutical example 3]

Manufacture of injections:

200 mg of glycyrrhizin are dissolved in a physiological saline solution to produce 100 ml.

[Pharmaceutical example 4]

Manufacture of injections:

200 mg glycyrrhizin, 2000 mg glycine and 100 mg cysteine are dissolved in a physiological saline solution to produce 100 ml.

The amount of active ingredient in these illustrative pharmaceutical examples may be varied to achieve the dosage unit range set forth above, and the amounts and nature of the inert pharmaceutical carrier ingredients may be varied to meet particular requirements. While the present invention has been illustrated with the aid of certain specific embodiments thereof, it will be readily apparent to others skilled in the art that the invention is not limited to these particular embodiments, and that departing from the spirit of the invention or the scope of the appended claims.

Certain pharmacological data of action of glycyrrhizin on the inhibition of growth of AIDS virus will be given hereinafter.

From these data, it has become apparent that glycyrrhizin an effective substance of licorice can strongly inhibit the growth of HIV and moreover, inhibit HIV induced cytopathic effect.

However, it has not shown any effect as the inhibition of reverse transcriptase effect, which has heretofore been prevented by the so-called conventional method.

In this case, glycyrrhizin seems to have a unique mode of action. On the other hand, with HIV hitherto, there has been no appropriate quantitative assay. Recently, by using MT-4 cells which are HTLV-I carrier cells, there has been established a quantitative method of plaque formation of HIV (Harade et al; Science 229, p.563-566, 1985). On the basis of such quantitative analysis, the inventor carried out certain experiments. Moreover, the inventor used Molt-4 clone No.8 (Kikukawa R. et al; J Virology 57 p.1159-1163, 1986) easily tending to cause cell fusion with the infection of HIV due to subclone of Molt-4 cells, thereby examining the effect of glycyrrhizin on the inhibition of cell fusion. Subsequently, in order to examine the effect and mechanism of glycyrrhizin on inhibiting of infected cells, the inventor proceeded to measure its effect on the activity of reverse transcriptase and neutralization of virus too.

(Pharmacological data)

Material and Methods:

1. Cells

HTLV-I carrying cell line, MT-4, and cloned cell of human leukemic T-cell line Molt-4, Molt-4 clone No.8, were used for this study. The cells were maintained in RPMI-1640 medium supplemented with 10% fetal calf serum, 100 IU/ml of penicillin and 100 μg/ml of streptomycin at 37°C in a $CO_2$ incubator.

2. Drugs

Preparation of aqueous solution of glycyrrhizin:

(a) Use of glycyrrhizin;

2 g of pure glycyrrhizin (manufactured by Minophagen Co., Ltd) were suspended in 90 ml of water while stirring and a little amount of ammonia T. S. or sodium hydroxide T.S. or potassium hydroxide T.S. at room temperature were added until they were dissolved perfectly. After adjusting pH to 6.5- 7.0, water was added at room temperature to produce 100 ml.

(b) Use of ammonium glycyrrhizinate;

90 ml of water were added to 2.04 g of ammonium glycyrrhizinate and after adjusting pH of the above resulting solution to 6.5-7.0 with ammonia T.S., water was added to produce 100 ml.

(c) Use of sodium glycyrrhizinate;

90 ml of water were added to 2.05 g of sodium glycyrrhizinate and after adjusting of pH of the above resulting solution to 6.5-7.0 with sodium hydroxide T.S., water was added to produce 100 ml.

(d) Use of potassium glycyrrhizinate;

2.09 g of potassium glycyrrhizinate were treated with potassium hydroxide T.S. in the same manner as above.

An aqueous solution of 2% glycyrrhizin (20 mg of glycyrrhizin contained in 1 ml) could be obtained by the aforesaid procedure.

At the time of its actual testing, it was suitably diluted with 0.01 M of a buffer solution of phosphoric acid until pH was adjusted to 7.2.

Preparation of 3'-azido-3'-deoxythymidine:

A nucleoside analogue, 3'-azido-3'-deoxythymidine was prepared essentially as described by Glinski et al. (J.Org.Chem. 38,p.4299-4305, 1973) and was phosphorylated by phosphorus oxychloride in triethyl phosphate according to the method of Yoshikawa et al. (Bull. Chem. Soc. Japan42,p.3505-3508, 1969).

3.Virus and virus infection

HIV was obtained from the culture supernatant of Molt-4/HIV. Titer of virus preparation was $6x10^4$ plaque forming units/ml. Infection of MT-4 cells and clone No. 8 cells of Molt-4 with HIV was made at a multiplicity of infectin of 0.002.

Briefly, the cells were mixed with virus solution and incubated for 1 hour at 37°C. After adsorption, infected cells were washed and resuspended in fresh medium to make concentration of $3 \times 10^5$ cells/ml and cultured in the presence or absence of various concentrations of glycyrrhizin in a $CO_2$ incubator.

4. Assay for HIV induced cytopathic effects

HIV induced cytopathic effects were analyzed by decrease in number of viable cells in HIV infected MT-4 or clone No. 8 cells of Molt-4. The viable cells were detected by trypan blue exclusion staining method.

5. Assay for HIV specific antigen expression

Virus-specific antigen expression in HIV infected MT-4 cells or clone No.8 cells of Molt-4 was determined by the indirect immunofluorescence method. Briefly, methanol fixed cells were incubated with 1:1000 diluted seropositive anti-HIV human serum (immunofluorescence titer 1:4096) for 30 min. at 37°C.

Then, the fixed cells were washed for 15 min. with phosphate buffered saline. The fluorescein-isothiocyanate conjugated rabbit anti-human IgG (Dakoppatts A/S, Copenhage, Denmark) was applied, incubated for 30 min. at 37°C and washed again with phosphate buffered saline. More than 500 cells were counted under a fluorescence microscope and the percentage of immunofluorescence-positive cells were calculated.

6. Plaque forming assay

To evaluate the direct effect of glycyrrhizin on the infection of HIV in MT-4 cells, plaque assay using agarose overlay medium with various concentrations of glycyrrhizin was performed. Briefly, in order to fasten MT-4 cells onto culture vessels, 35 mm polystyrene tissue culture dishes were coated with poly-L-lysine (Sigma Chemical Co., St. Louis,Mo) One and half milliliters of $1.50 \times 10^6$/ml MT-4 cells were dropped onto each poly-L-lysine-coated dish and incubator for 1 hour at room temperature. The dishes were gently washed with phosphate buffered saline to remove unbound cells, 100 μ1 of appropriate diluted virus preparation was slowly added and incubated for 1 hour at room temperature for virus adsorption. After adsorption, 1 ml of the agarose overlay medium consisting of RPMI 1640 medium with 10 % fetal calf serum, antibiotics and 0.6 % agarose (Sea plaque Agarose, Marine Colloid Corp., Rockland, Me)

was poured into each dish. The dishes were incubated in a $CO_2$ incubator at 37°C for 3 days and 1 ml of agarose overlay medium containing neutral red was added. The dishes were incubated 3 more days and visible plaque were counted. All experiments were carried out in triplicate.

7. Reverse transcriptase assay

Supernatants of Molt-4/HIV were concentrated 100 times by sucrose gradient ultracentrifugation. The viral pellets were disrupted by addition of suspension buffer containing 5 mM Tris-HCl(pH 8.1), 0.5 M KCl, 0.1 mM dithiothreitol and 0.1 % Triton® X-100. The assay for reverse transcriptase activity was performed at 37°C for 1 h with a 10 µl of the disrupted HIV in a final volume of 50 µl containing 50 mM Tris-HCl(pH 8.4), 2 mM dithiothreitol, 100 mM KCl, 10 mM MgCl, 0.01 % Triton X-100. 1 µCi $^3$H-thymidine triphosphate (57 Ci/m mol.; Amersham, Buckinghanshire, UK) and 50 µg/ml poly(rA)/oligo(dT)(P-L Biochemicals Inc., Milwaukee, Wis). The reaction was stopped with 5% trichloroacetic acid, and precipitates were collected on glass fibre filters and counted in a liquid scintillation counter (Harada et. al; Virology 146, p.272-281,1985b). The assays were carried out in triplicate.

8. $^3$H-thymidine uptake in MT-4 cells or normal pheripleral blood monouclear cells.

MT-4 cells were adjusted to $3x10^5$ cells/ml in fresh medium and cultured with various concentrations of glycyrrhizin in a 96-flat bottom well microtiter plate for 3 days. $10^6$ pheripleral blood monouclear cells were cultured with 1 µg/ml of poly clonal mitogen PHA or concanavalin A various concentrations of glycyrrhizin for 3 days. Cells were incubated with either 1 µCi/well of $^3$H-thymidine (20 Ci/m mol.; New Engl.Nuclear, Boston, MA), for the last 18 hours. The cells were harvested on a glassfiber filter paper and counted in a liquid scintillation counter. All experiments were carried out in triplicate.

9. Inactivation of HIV particles by glycyrrhizin

0.5 ml of HIV containing plaque forming units was mixed with 0.5 ml of various concentrations of glycyrrhizin in the test tubes and incubated at 37°C.

After 60 min. incubation 0.1 ml of each mixture was diluted with 19.9 ml of culture medium, and 0.1 ml of the 20-fold diluted virus preparation was added. After adsorption, 1 ml of the agarose overlay medium consisting of RPMI-1640 medium with 10 % fetal calf serum, antibiotics and 0.6 % agarose was poured in. The maximum concentration of the diluted glycyrrhizin in plaque assay medium was 0.01 mg/ml, and this concentration itself reduced about 10 % of HIV induced plaque forming activity in MT-4 cells.

Result:

1. Suppression of HIV induced plaque forming activity by glycyrrhizin

We first examined the effect of glycyrrhizin on HIV induced plaque forming activity in MT-4 cells by using agarose overlay medium which directly contains various concentrations of glycyrrhizin. Glycyrrhizin suppressed 34.2 % of plaque formation at a concentration of 0.05 mg/ml and increased the degree of inhibition in a dose dependent manner. Glycyrrhizin completely inhibited the plaque formation at the concentration of 0.5 and 1 mg/ml. The ID50 of glycyrrhizin was 0.125 mg/ml (Fig.1). The ID50 of glycyrrhizin against plaque formation of this virus was lower than that of glycyrrhizin against herpes simplex virus (0.60 mg/ml) or Varicella-Zoster virus (0.58 mg/ml) in human embryonic fibroblast.

2. Inhibition of HIV induced cytopathic effects by glycyrrhizin

We next examined the influence of cytopathic effect of HIV infection of MT-4 by glycyrrhizin in the suspension culture condition without agarose (Fig.2). Upon HIV infection, the viable MT-4 cells were decreased and under the present experimental condition (multiplicity of infection:0.002) the number of viable cells was $3.2x10^5$ cells/ml while the uninfected MT-4 cells increased to $12.0 \times 10^5$ cells/ml on day 3 after infection. Meanwhile, when 0.25, 0.5 or 1 mg/ml of glycyrrhizin was added, the growth inhibition of infected cultures was not significant as compared with the uninfected MT-4 cells.

Moreover, even higher concentrations of glycyrrhizin were not so toxic to the cells; e.g. at a concentration of 2 mg/ml glycyrrhizin caused 50 % growth inhibition, but it did not kill the cells. These results suggest that glycyrrhizin has a protective effect against the HIV induced cytopathic effect in the concentration range, in which glycyrrhizin does not inhibit the growth of MT-4 cells. By $^3$H-thymidine up take methods, the ID50 of glycyrrhizin for DNA synthesis of uninfected MT-4 cells was shown to be 2.15 mg/ml, and the selectivity index (the ratio on the ID50 for host cell DNA synthesis to the average ID50 for HIV) was 17.2. These results indicated that glycyrrhizin inhibited the replication of HIV selectively in MT-4 cell cultures.

3. Effect of glycyrrhizin on the expression of HIV-specific antigen

When MT-4 cells were infected with HIV, 73.5 % of the cells became positive for virus antigen on day 3 after infection, as determined by an indirect immunofluorescence (IF) technique (Table I). A significant inhibition was observed when the HIV infected MT-4 cells were cultured in the presence of 0.25 mg/ml glycyrrhizin. At the concentrations of 0.5 and 1 mg/ml, the antigen expression was almost completely suppressed.

Table I

Inhibitory effect of glycyrrhizin on the expression of HIV specific antigen

| conc. of glycyrrhizin (mg/ml) | percentage of IF-positive cells |
|---|---|
| 1 | 0.6 |
| 0.5 | 1.0 |
| 0.25 | 23.3 |
| 0.05 | 69.3 |
| 0.005 | 70.9 |
| 0 | 73.9 |

More than 500 cells were counted and percentage of immunofluorescence-positive cells was calculated on day 3 after infection.

4. Effect of glycyrrhizin on inactivation of HIV particles

It is known that glycyrrhetinic acid does not only suppress the replication of herpes simplex virus and VSV, but also inactivates the virus particles, when these viruses are incubated with this compound for 30 min. However, in our experiments, glycyrrhizin did not inactivate HIV particles even at a concentration of 2 mg/ml.

5. Effect of glycyrrhizin on reverse transcriptase activity of HIV

Table II shows the effect of glycyrrhizin and 3′-azido-3′-deoxythymidine triphosphate compound against the reverse transcriptase activity of HIV in vitro. 3′-azido-3′-deoxythymidine shows strong inhibition of reverse transcriptase as triphosphate, even 0.005 mg/ml azido-TTP inhibited 97 % of reverse transcriptase activity. However, glycyrrhizin did not inhibit reverse transcriptase activity of HIV, even at a concentration of 10 mg/ml.

Table II

Effect of glycyrrhizin and 3′-azido-3′-deoxythymidine-TTP on the reverse transcriptase(RT) activity of HIV

| conc. of drug (mg/ml) | RT activity [a]($\times 10^{-4}$ cpm) | |
|---|---|---|
| | glycyrrhizin | 3′-azido-TIP |
| 10 | 270±21.4 | - |
| 5 | 297±17.1 | - |
| 1 | 247± 3.0 | 0.8±0.2 |
| 0.5 | 264± 2.8 | 0.7±0.1 |
| 0.1 | 241±14.5 | 1.0±0.3 |
| 0.05 | - | 1.3±0.3 |
| 0.005 | - | 7.9±1.5 |
| 0.0005 | - | 56.5±0.6 |
| 0 | 256±14.2 | 155 ±9.7 |

a. Experiments were carried out in triplicate.
Number represents the mean ± standard deviation.

6. Effect of glycyrrhizin on normal lymphocyte blastformation

We tested the effect of glycyrrhizin on lectin-induced reactivity of normal lymphocytes. The ID50 of glycyrrhizin on PHA and concanavalin A response was 2.1 and 2.0 mg/ml, respectively.

7. Inhibitory effect of glycyrrhizin on fusion sand cytopathy of HIV infected clone No.8 cells of Molt-4.

As Mt-4 cells had extreme sensitivity to the cytopathic effect of HIV and were destroyed in a few days, we used cloned Molt-4 cell line instead of MT-4 cells. The clone No.8 cells of Molt-4 are also sensitive to the virus, fuse to each other easily and form giant cells but the induction time of the cytopathic effect is much longer than of MT-4 cells. We added glycyrrhizin (1 mg/ml) to HIV infected clone No.8 cells of Molt-4 and observed the cytopathic effect on day 3, 6 and 9. Table III shows that on day 3 cell viability of glycyrrhizin-treated and untreated groups was 91.4 % and 89.8 %, respectively, and immunofluorescence positive cells of both groups were less than 4.68 %. On day 6, there was no difference in cell viability of any groups, but immunofluorescence positive cells increased in the untreated group. On day 9, cell viability of the untreated group decreased to 38.6 %, but that of the glycyrrhizin treated group was 84.4 %. Immunofluorescence(IF) positive cells were 99% in untreated group, but it was still 5.8 % in glycyrrhizin-treated group. Such difference between drug treated and untreated cultures were also expressed as that of reverse transcriptase activity and number of HIV in the culture fluids, especially on day 9. Although the fusion on clone No.8 cells of Molt-4 was observed on day 6 and 9, in the glycyrrhizin-treated cells the phenomenon did not occur.

Table III

Effect of glycyrrhizin on cell viability and HIV-specific antigen expression of Molt-4 clone No.8 cells.

| | glycyrrhizin concentration | |
|---|---|---|
| | 0 | 1 mg/ml |
| on day 3 | | |
| viable cells ($\times 10^5$/ml) | 18.1 | 15.0 |
| viability (%) | 89.8 | 91.4 |
| IF positive cells(%) | 4.7 | 3.0 |
| RT (cpm) | 1470 | 1536 |
| number of HIV (pfu/ml) | 500< | 500< |
| on day 6 | | |
| viable cells ($\times 10^5$/ml) | 42 | 34.0 |
| viability (%) | 82.4 | 83.4 |
| IF posivive cells(%) | 11.8 | 7.1 |
| RT (cpm) | 10624 | 5369 |
| number of HIV (pfu/ml) | 2500 | 500< |
| on day 9 | | |
| viable cells ($\times 10^5$/ml) | 37.8 | 75.7 |
| viability (%) | 38.6 | 84.4 |
| IF positive cells(%) | 99.0 | 5.8 |
| RT (cpm) | 20344 | 4369 |
| number of HIV (pfu/ml) | 15500 | 1000 |

HIV-infected clone No.8 cells of Molt-4 ($3\times 10^5$ cells/ml) were cultured with or without 1 mg/ml of glycyrrhizin. Cells numbers, viability, percentage of immunofluorescence-positive cells, reverse transcriptase activity and number of HIV were counted on day 3, 6 and 9. reverse transcriptase activities in culture fluids were expressed as CPM per $10^4$ viable cells. Numbers of HIV in the culture fluids were counted by using the MT-4 cell plaque assay system.

(Toxicity)

Acute toxicity of glycyrrhizin:

$LD_{50}$ of glycyrrhizin is low as mentioned below.

1) Oral administration (rat) 3.0 g/kg
2) Subcutaneous injection 5 % aqueous solution (mouse) 1873.3 mg/kg
3) Intravenous injection 2 % aqueous solution (mouse) 682.5 mg/kg
4) Intraperitoneal injection 0.2 % aqueous solution (mouse) 225-244 ml/kg

**Claims**

1) The use of glycyrrhizin represented by the following formula :

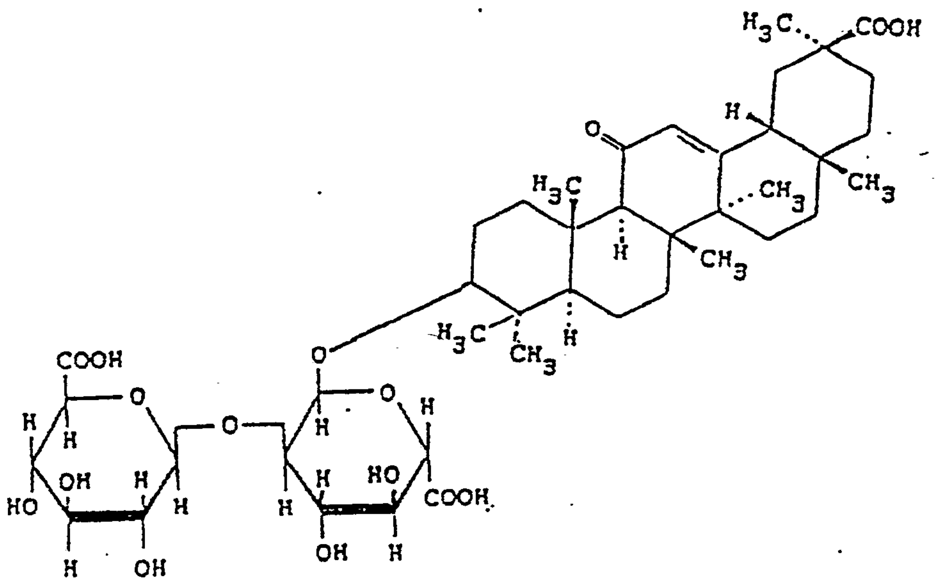

and its pharmaceuticcaly acceptable alkali-added salts,for the manufacture of a medicament for prophylaxis and treatment of acquired immune deficiency syndrome (AIDS).

2) The use as claimed in claim 1, wherein the agent is formed in tablets suitable for oral administration in the treatment of AIDS.

3) The use as claimed in claim 2, wherein the form of doses is formed in tablets, granules, capsules and powder, for the treatment of AIDS.

4) The use as claimed in claim 1 wherein the agent is formed as suitable for non-oral administration,for the treatment of AIDS.

5) The use as claimed in claim 4, wherein the agent is provided in injectable form, for the treatment of AIDS.

**Patentansprüche**

1. Verwendung von Glycyrrhizin, dargestellt durch die folgende Formel:

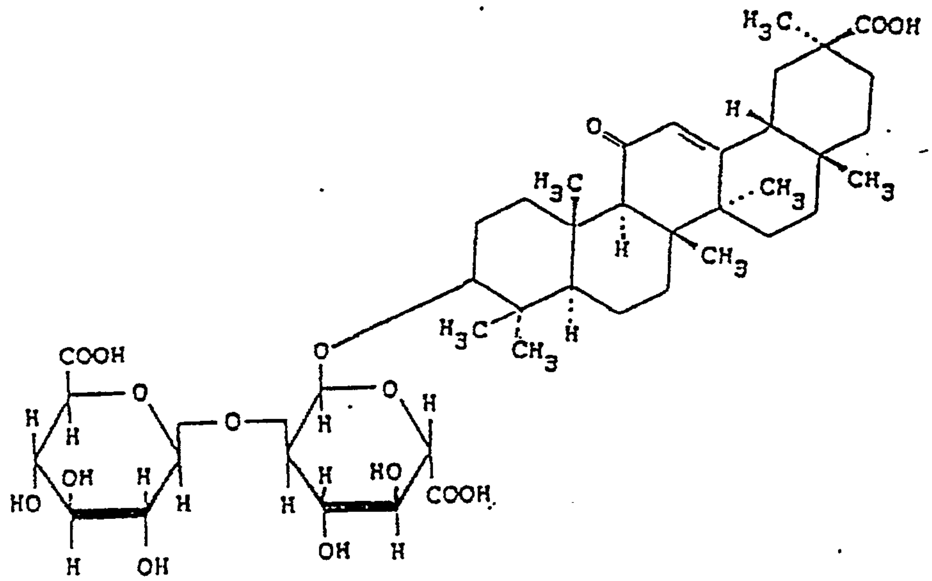

und seinen pharmazeutisch annehmbaren alkalizugesetzten Salzen für die Herstellung eines Medikaments zur Prophylaxe und Behandlung des Immunschwäche-(AIDS)-Syndroms.

2. Verwendung nach Anspruch 1, worin das Mittel in Tablettenform gebildet wird, die für die orale Verabreichung bei der Behandlung von AIDS geeignet ist.

3. Verwendung nach Anspruch 2, worin die Darreichungsform durch Tabletten, Granulat, Kapseln und Pulver für die Behandlung von AIDS gebildet wird.

4. Verwendung nach Anspruch 1, worin das Mittel als für die nicht-orale Verabreichung bei der Behandlung von AIDS geeignet, gebildet wird.

5. Verwendung nach Anspruch 4, worin das Mittel in injizierbarer Form für die Behandlung von AIDS bereitgestellt wird.

**Revendications**

1. Utilisation de glycyrrhizine représentée par la formule suivante:

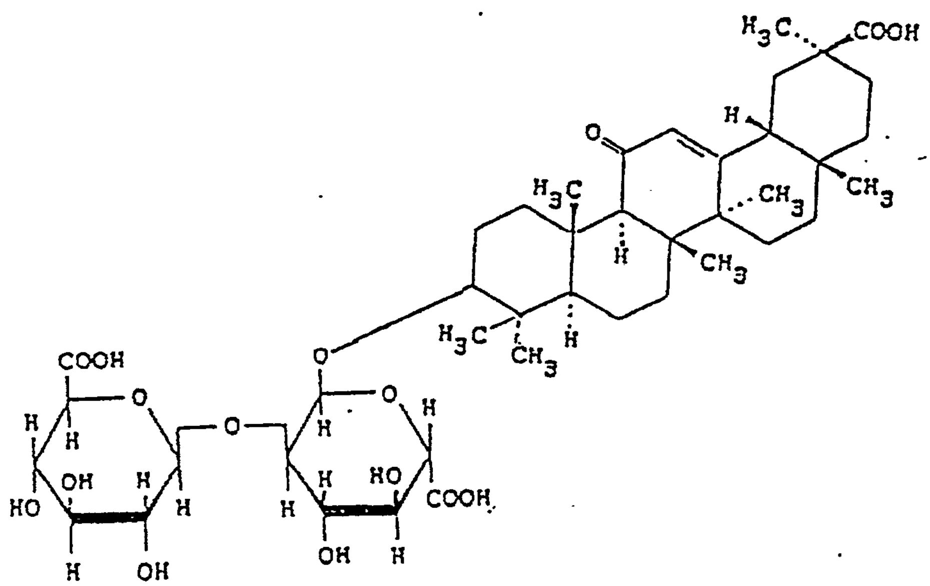

et de ses sels formés par addition de substances basiques, pharmaceutiquement acceptables, dans la fabrication d'un médicament pour la prophylaxie et le traitement du syndrome immunodéficitaire acquis (SIDA).

2. Utilisation selon la revendication 1, dans laquelle l'agent est mis sous forme de comprimés propres à l'administration orale dans le traitement du SIDA.

3. Utilisation selon la revendication 2, dans laquelle les unités posologiques sont mises sous forme de comprimés, de granules, de capsules et de poudre, pour le traitement du SIDA.

4. Utilisation selon la revendication 1, dans laquelle l'agent est mis sous une forme convenant pour l'administration non orale, pour le traitement du SIDA.

5. Utilisation selon la revendication 4, dans laquelle l'agent est mis sous forme injectable, pour le traitement du SIDA.

FIG.1

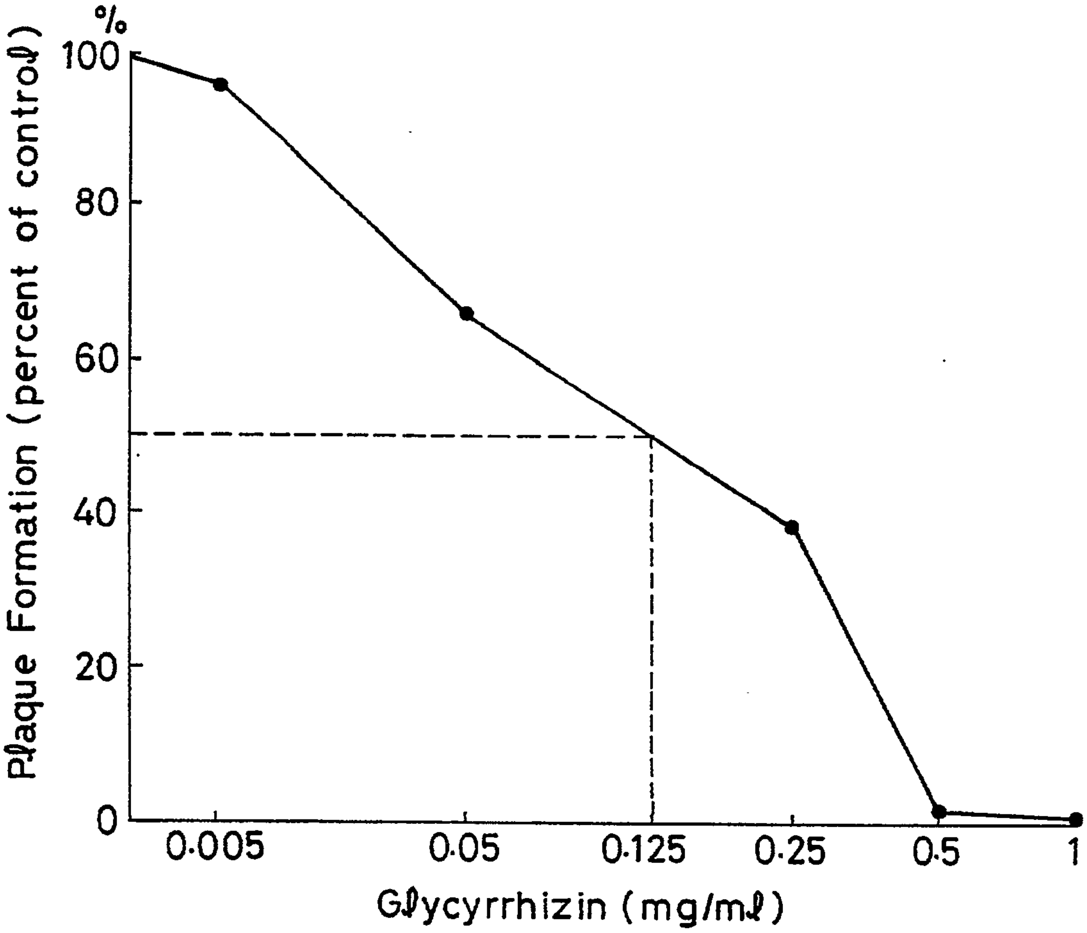
%
100
80
60
40
20
0
Plaque Formation (percent of control)
0.005
0.05
0.125
0.25
0.5
1
Glycyrrhizin (mg/ml)

FIG. 2

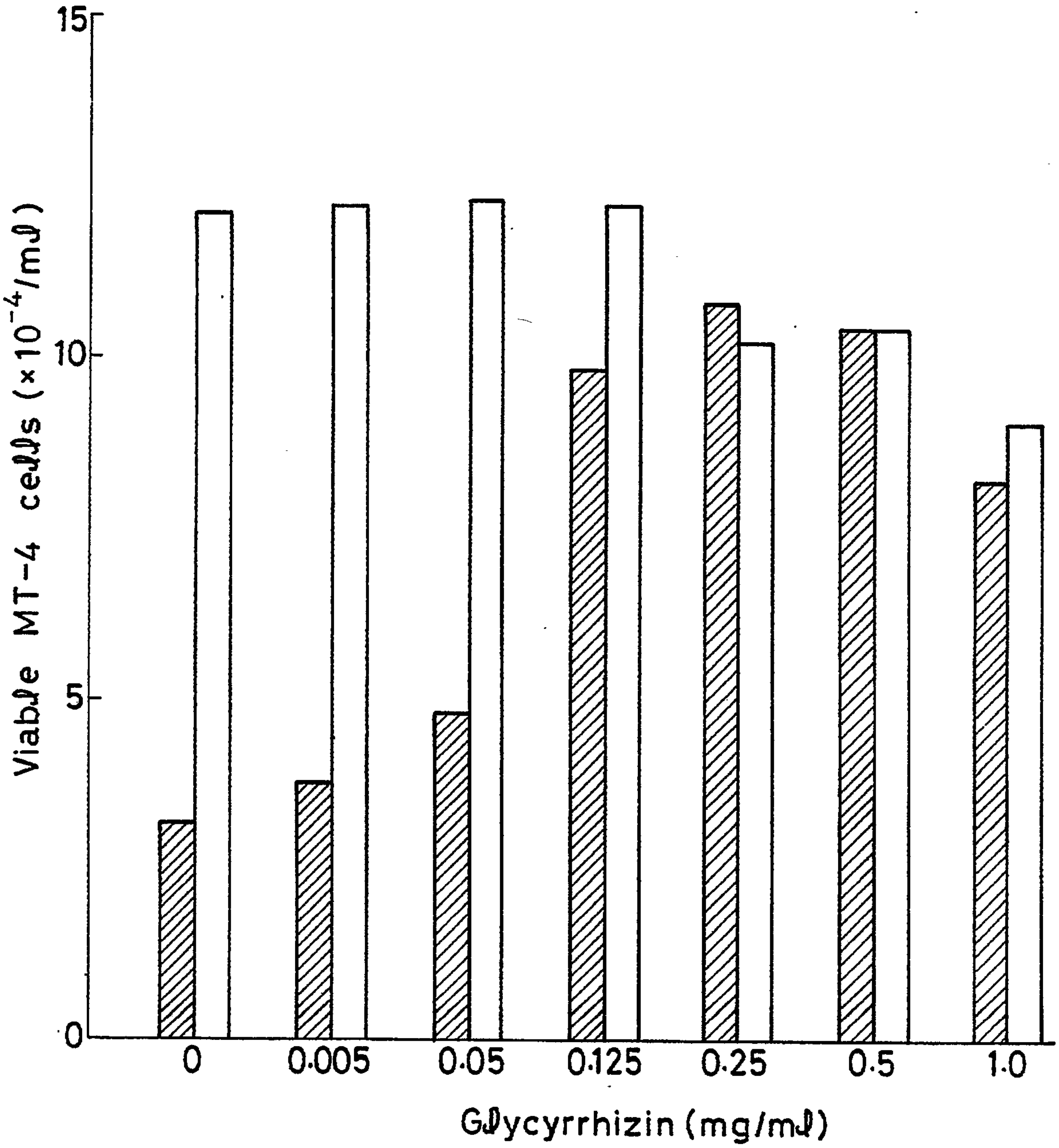
15
10
5
0
Viable MT-4 cells (×10⁻⁴/ml)
0
0.005
0.05
0.125
0.25
0.5
1.0
Glycyrrhizin (mg/ml)